(19) Europäisches Patentamt
European Patent Office
Office européen des brevets

(11) Veröffentlichungsnummer: **0 018 542**
**B1**

(12) **EUROPÄISCHE PATENTSCHRIFT**

(45) Veröffentlichungstag der Patentschrift: **02.03.83**

(51) Int. Cl.³: **C 07 D 417/06,** A 61 K 31/54

(21) Anmeldenummer: **80102033.0**

(22) Anmeldetag: **16.04.80**

(54) **Phenothiazinderivate, diese enthaltende pharmazeutische Zubereitungen und Verfahren zu ihrer Herstellung.**

(30) Priorität: **02.05.79 DE 2917650**

(43) Veröffentlichungstag der Anmeldung: **12.11.80 Patentblatt 80/23**

(45) Bekanntmachung des Hinweises auf die Patenterteilung: **02.03.83 Patentblatt 83/9**

(84) Benannte Vertragsstaaten: **AT BE CH DE FR GB IT LI NL SE**

(56) Entgegenhaltungen:
**FR-A-1 060 715**
**FR-A-1 488 281**

**DIE PHARMAZIE, Band 31, Januar 1. 1976, V.E.B. Verlag, Berlin, DE ABU-SHADY, H., et al. »Synthesis of some new phenothiazine derivatives of expected medicinal value«, Seiten 18—20**

**Die Akte enthält technische Angaben, die nach dem Eingang der Anmeldung eingereicht wurden und die nicht in dieser Patentschrift enthalten sind.**

(73) Patentinhaber: **Merck Patent Gesellschaft mit beschränkter Haftung, Frankfurter Strasse 250, D-6100 Darmstadt (DE)**

(72) Erfinder: **Gante, Joachim, Dr.Chem, Stormstrasse 4, D-6100 Darmstadt-Arheiligen (DE)**
Erfinder: **Radunz, Hans-Eckart, Dr.-Chem., Hügelstrasse 13, D-6109 Mühltal 2 (DE)**
Erfinder: **Orth, Dieter, Dr.-Chem., Jahnstrasse 83, D-6100 Darmstadt (DE)**
Erfinder: **Schliep, Hans-Jochen, Dr., Weingartenstrasse 16, D-6109 Mühltal-2 (DE)**
Erfinder: **Schorscher, Ernst, Dr., Im Fiedlersee 1, D-6100 Darmstadt-Arheilgen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

# Phenothiazinderivate, diese enthaltende pharmazeutische Zubereitungen und Verfahren zu ihrer Herstellung

Die Erfindung betrifft neue Phenothiazinderivate der allgemeinen Formel I

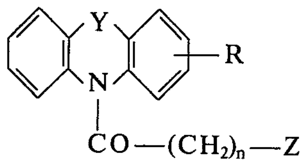

(I)

worin

R H, F, Cl, Br, J, $CH_3$, $CF_3$, CN, $CH_3O$ oder $CH_3CO$,
Y S, SO oder $SO_2$,
Z 1-Imidazolyl, 2-Methyl-1-imidazolyl, 1-Pyrazolyl oder 1-Benzimidazolyl und
n 1, 2 oder 3 bedeuten,

und deren physiologisch unbedenkliche Säureadditionssaize.

Der Erfindung lag die Aufgabe zugrunde, neue Verbindungen aufzufinden, die zur Herstellung von Arzneimitteln verwendet werden können. Diese Aufgabe wurde durch die Bereitstellung der Verbindungen der allgemeinen Formel I gelöst.

Es wurde gefunden, daß die Verbindungen der allgemeinen Formel I und ihre physiologisch unbedenklichen Säureadditionssalze bei guter Verträglichkeit wertvolle pharmakologische Eigenschaften besitzen. So zeigen sie beispielsweise Wirkungen auf den Kreislauf, insbesondere blutdrucksenkende, ferner auch diuretische Wirkungen.

Die Substanzen vermindern z. B. den an der Carotisschlinge des wachen mischrassigen Hundes gemessenen Blutdruck [Methodik vgl. E. C. van LEERSUM, Pflüger's Archiv 142, 377 – 395 (1911)] a) bei neurogen hypertonen Tieren [Methodik vgl. K. S. GRIMSON, Archives Surgery 43, 284 – 305 (1941)] sowie b) bei nephrogen hypertonen Tieren [Methodik vgl. I. H. PAGE, Science 89, 273 – 274 (1939)] im 10-Tage-Dauerversuch bei oraler Gabe von Dosen, die niedriger als 2,0 mg/kg liegen können, dosisabhängig auf ein erniedrigtes Niveau.

Weiterhin wird der bei Ratten plethysmographisch am Schwanz der wachen Tiere gemessene Blutdruck [Methodik vgl. M. GEROLD und H. TSCHIRKY, Arzneimittelforschung 18, 1285 – 1287 (1968)] während einer 4tägigen oralen Behandlung mit einmal täglicher Substanzgabe ebenfalls auf ein geringeres Niveau abgesenkt, und zwar a) bei spontan hypertonen Ratten (Stamm SHR/NIH – MO/CHB – EMD) wie auch b) bei DOCA-Salz-hypertonen Ratten (Methodik vgl. M. GEROLD und H. TSCHIRKY, l.c.).

Die als Funktionstest durchgeführte Prüfung der Blutdruckreaktion nach intravenöser Gabe von Noradrenalin an 4tägig oral vorbehandelten und zur Testung dann demedullierten normotonen Ratten [Methodik vgl. R. E. SHIPLEY und J. H. TILDEN, Proc. Soc. Exper. Biol. Med. 64, 453 – 455 (1947)] ergab eine verminderte Reaktivität des Gefäßsystems [vgl. J. KRAETZ et al., Naunyn-Schmiedeberg's Archives of Pharmacol. Supplem. to Vol. 302, R 42 (1978)]. Die Bestimmung der diuretischen Wirkung der Verbindungen kann an Ratten unter Berücksichtigung der Angaben von W. L. LIPSCHITZ et al., J. Pharmacol. exp. Ther. 79, 97 – 110 (1943), erfolgen.

Die Verbindungen der allgemeinen Formel I und ihre physiologisch unbedenklichen Säureadditionssalze können daher als Arzneimittelwirkstoffe in der Human- und Veterinärmedizin und auch als Zwischenprodukte zur Herstellung anderer Arzneimittelwirkstoffe verwendet werden.

Gegenstand der Erfindung sind die Verbindungen der allgemeinen Formel I und deren physiologisch unbedenkliche Säureadditionssalze.

Ähnliche Verbindungen sind bekannt. Beispielsweise ist in der FR-PS 10 60 715 eine allgemeine Formel angegeben, die der vorliegenden allgemeinen Formel I (Y = S) weitgehend entspricht, an Stelle des Restes Z jedoch einen sekundären oder tertiären Aminorest enthält, der u. a. auch von einem heterocyclischen Amin abgeleitet sein kann; als derartige Reste sind nur Piperidino und Pyrrolidino genannt. Die dort beschriebenen Verbindungen sollen spasmolytische, antihistaminische oder antiinflammatorische Eigenschaften besitzen. Weiterhin sind in der FR-PS 14 88 281 ähnliche Verbindungen beschrieben, die anthelmintisch wirksam sind; unter die dort angegebene allgemeine Formel fallen auch Substanzen, die der allgemeinen Formel I (R = H, Y = S) entsprechen, aber an Stelle des Restes Z einen in 2-Stellung durch einen aromatischen oder heteroaromatischen Rest substituierten 1-Benzimidazolylrest tragen.

An keiner Stelle kann diesen Druckschriften jedoch ein Hinweis auf die erfindungsgemäßen Verbindungen der allgemeinen Formel I entnommen werden, insbesondere auch nicht ein Hinweis auf ihre wertvollen blutdrucksenkenden und diuretischen Eigenschaften.

In der allgemeinen Formel I steht der Rest R bevorzugt in 2-Stellung; er kann jedoch auch in 1-, 3-oder 4-Stellung stehen. Der Rest Y ist bevorzugt S oder SO. Der Rest Z steht vorzugsweise für eine 1-Imidazolylgruppe. Der Parameter n hat bevorzugt den Wert 2.

Dementsprechend sind Gegenstand der Erfindung insbesondere diejenigen Verbindungen der allgemeinen Formel I, in denen mindestens einer der genannten Reste eine der vorstehend angegebenen bevorzugten Bedeutungen hat.

Gegenstand der Erfindung ist ferner ein Verfahren zur Herstellung der Verbindungen der allgemeinen Formel I sowie ihrer physiologisch unbedenklichen Säureadditionssalze, dadurch gekennzeichnet, daß man ein Phenothiazinderivat der allgemeinen Formel II

$$Phe-CO-(CH_2)_n-X \qquad (II)$$

worin

Phe den Rest

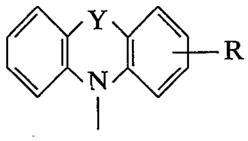

und

X Cl, Br, J, OH oder reaktionsfähig verestertes OH bedeutet und

R, Y und n die angegebene Bedeutung haben

mit einer Base der allgemeinen Formel III

$$H-Z \qquad (III)$$

worin

Z die angegebene Bedeutung hat

oder daß man ein Penothiazinderivat der allgemeinen Formel IV

$$Phe-H \qquad (IV)$$

worin

Phe die angegebene Bedeutung hat

mit einer Carbonsäure der allgemeinen Formel V

$$HOOC-(CH_2)_n-Z \qquad (V)$$

worin

n und Z die angegebenen Bedeutungen haben,

oder einem ihrer funktionellen Derivate umsetzt und daß man gegebenenfalls durch Behandeln mit oxydierenden Mitteln ein so erhaltenes Phenothiazin der allgemeinen Formel I (Y=S) zum Sulfoxid oder Sulfon der allgemeinen Formel I (Y=SO oder $SO_2$) oder ein so erhaltenes Sulfoxid der allgemeinen Formel I (Y=SO) zum Sulfon der allgemeinen Formel I (Y=$SO_2$) oxydiert und/oder eine so erhaltene Base der allgemeinen Formel I durch Behandeln mit einer Säure in eines ihrer physiologisch unbedenklichen Säureadditionssalze umwandelt.

Die Herstellung der Verbindungen der allgemeinen Formel I erfolgt im übrigen nach an sich bekannten Methoden, wie sie in der Literatur (z. B. in den Standardwerken wie Houben-Weyl, Methoden der Organischen Chemie, Georg-Thieme-Verlag, Stuttgart; Organic Reactions, John Wiley & Sons, Inc., New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe der allgemeinen Formeln II bis V können gewünschtenfalls auch in situ gebildet werden, derart, daß man sie aus dem Reaktionsgemisch nicht isoliert, sondern sofort weiter zu den Verbindungen der allgemeinen Formel I umsetzt.

Die Verbindungen der allgemeinen Formel I werden vorzugsweise durch Umsetzung von Phenothiazinderivaten der allgemeinen Formel II mit heterocyclischen Basen der allgemeinen Formel

III erhalten.

In den Phenothiazinderivaten der allgemeinen Formel II ist der Rest X vorzugsweise Cl oder Br; er kann jedoch auch J, OH oder eine reaktionsfähig funktionell abgewandelte OH-Gruppe bedeuten, insbesondere Alkylsulfonyloxy mit 1–6 (z. B. Methansulfonyloxy) oder Arylsulfonyloxy mit 6–10 C-Atomen (z. B. Benzolsulfonyloxy, p-Toluolsulfonyloxy, 1- oder 2-Naphthalinsulfonyloxy).

Die Ausgangsstoffe der allgemeinen Formeln II und III sind teils bekannt, teils neu. Die nicht bekannten unter diesen Verbindungen können leicht analog zu den bekannten Verbindungen hergestellt werden. So sind die Phenothiazinderivate der allgemeinen Formel II durch Acylierung von Phenothiazinen der allgemeinen Formel Phe–H (IV) mit Carbonsäuren der allgemeinen Formel $HOOC-(CH_2)_nX$ oder ihren funktionellen Derivaten erhältlich.

Die Umsetzung der Verbindungen der allgemeinen Formeln II und III verläuft nach Methoden, wie sie für die N-Alkylierung von Imidazolen, Pyrazolen oder Benzimidazolen aus der Literatur bekannt sind. So kann man z. B. in Abwesenheit eines Lösungsmittels die Komponenten miteinander verschmelzen, gegebenenfalls im geschlossenen Rohr oder im Autoklav. Es ist aber auch möglich, die Verbindungen in Gegenwart eines indifferenten Lösungsmittels miteinander umzusetzen. Als Lösungsmittel eignen sich z. B. Kohlenwasserstoffe wie Benzol, Toluol, Xylol; Ketone wie Aceton, Butanon; Alkohole wie Methanol, Äthanol, Isopropanol, n-Butanol; Äther wie Tetrahydrofuran oder Dioxan; Amide wie Dimethylformamid oder N-Methylpyrrolidon; Nitrile wie Acetonitril; gegebenenfalls auch Gemische dieser Lösungsmittel untereinander oder Gemische mit Wasser. Der Zusatz eines säurebindenden Mittels, beispielsweise eines Alkali- oder Erdalkalimetallhydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triäthylamin, Dimethylanilin, Pyridin oder Chinolin oder eines Überschusses der Base der allgemeinen Formel III kann günstig sein. Weiterhin kann man z. B. Chlorverbindungen der allgemeinen Formel II (X = Cl) mit Basen der allgemeinen Formel III im Molverhältnis 1 : 1 in hochsiedenden Lösungsmitteln wie Toluol oder Xylol miteinander umsetzen, wobei Chlorwasserstoff abgespalten wird. Die Reaktionszeit liegt je nach den angewendeten Bedingungen zwischen einigen Minuten und 7 Tagen, die Reaktionstemperatur zwischen etwa 0 und 150° C, normalerweise zwischen 60 und 130° C.

Die Verbindungen der allgemeinen Formel I sind weiterhin erhältlich, indem man ein Phenothiazin-derivat der allgemeinen Formel IV mit einer Carbonsäure der allgemeinen Formel V oder einem ihrer funktionellen Derivate acyliert.

Die Ausgangsstoffe der allgemeinen Formeln IV und V sind ebenfalls weitgehend bekannt. Sofern sie nicht bekannt sind, können sie analog zu bekannten Verbindungen hergestellt werden. Beispielsweise kann man die Carbonsäuren der allgemeinen Formel V durch Umsetzung von Carbonsäuren der allgemeinen Formel $HOOC-(CH_2)_n-X$ mit Basen der allgemeinen Formel H–Z erhalten. Funktionelle Derivate der Carbonsäuren der allgemeinen Formel V sind vorzugsweise ihre Halogenide, insbesondere ihre Chloride und Bromide, ferner z. B. ihre Anhydride, die entsprechenden Säureazide und reaktionsfähige Ester. Man acyliert vorteilhaft in Lösung bzw. Suspension, wobei man als Lösungs- bzw. Suspensionsmittel eines oder mehrere der oben angegebenen verwenden kann. Ferner eignen sich als Lösungsmittel z. B. halogenierte Kohlenwasserstoffe wie 1,2-Dichloräthan oder Chlorbenzol; Ester wie Äthylacetat. Auch bei der Acylierung ist der Zusatz einer der oben angegebenen Basen mitunter vorteilhaft, insbesondere, wenn man ein Säurehalogenid oder Anhydrid der Carbonsäure der allgemeinen Formel V als Acylierungsmittel verwendet. Verwendet man dagegen die Carbonsäure selbst, so ist es zweckmäßig, ein Dehydratisierungsmittel zuzusetzen, beispielsweise ein Carbodiimid wie Dicyclohexylcarbodiimid. Die Acylierungstemperaturen liegen in der Regel zwischen –20° und +100° C, vorzugsweise zwischen –5° und +40° C. Die Reaktionszeiten können etwa zwischen 1 Stunde und 4 Tagen variieren.

Ein so erhaltenes Phenothiazin der allgemeinen Formel I (Y = S) kann, falls erwünscht, zum entsprechenden Sulfoxid oder Sulfon der allgemeinen Formel I (Y = SO oder $SO_2$) oder ein erhaltenes Sulfoxid der allgemeinen Formel I (Y = SO) kann zum entsprechenden Sulfon der allgemeinen Formel I (Y = $SO_2$) oxydiert werden.

Auch hierbei arbeitet man nach an sich bekannten Methoden; die Reaktionsbedingungen können im einzelnen aus der Literatur leicht entnommen werden. Will man z. B. die Sulfoxide erhalten, so oxydiert man beispielsweise mit Wasserstoffperoxid, Persäuren, Cr(VI)-Verbindungen wie Chromsäure, Salpetersäure, nitrosen Gasen, $N_2O_3$, Halogenen wie Chlor, Hypochloriten, $KMnO_4$, N-Bromsuccinimid, 1-Chlorbenztriazol, Ce(IV)-Verbindungen wie $(NH_4)_2Ce(NO_3)_6$, negativ substituierten aromatischen Diazoniumsalzen wie o- oder p-Nitrophenyldiazoniumchlorid oder elektrolytisch unter verhältnismäßig milden Bedingungen und bei relativ niedrigen Temperaturen (etwa –80 bis +100° C). Will man dagegen die Sulfone erhalten, so verwendet man die gleichen Oxydationsmittel unter kräftigeren Bedingungen und/oder im Überschuß und arbeitet in der Regel bei höheren Temperaturen. Bei diesen Umsetzungen können die üblichen inerten Lösungsmittel zugegen oder abwesend sein. Als inerte Lösungsmittel eignen sich beispielsweise Wasser, wässerige Mineralsäuren, wässerige Alkalilaugen, niedere Alkohole wie Methanol oder Äthanol, Ester wie Äthylacetat, Ketone wie Aceton, niedere Carbonsäuren wie Essigsäure, Nitrile wie Acetonitril, Kohlenwasserstoffe wie Benzol, chlorierte Kohlenwasserstoffe wie Chloroform oder $CCl_4$.

Ein bevorzugtes Oxydationsmittel ist 30%iges wässeriges Wasserstoffperoxid. Dieses führt bei Anwendung der berechneten Menge in Lösungsmitteln wie Essigsäure, Aceton, Äthanol oder wässeriger Natronlauge bei Temperaturen zwischen −20 und 100°C zu den Sulfoxiden, im Überschuß bei höheren Temperaturen, vorzugsweise in Essigsäure oder in einem Gemisch aus Essigsäure und Acetanhydrid, zu den Sulfonen.

Eine weitere Möglichkeit zur Herstellung der Sulfoxide besteht darin, daß man die Phenothiazine mit Chlor, z. B. in feuchtem Benzol oder in Essigsäure, behandelt. Die intermediär erhaltenen Dichlorverbindungen werden durch Hydrolyse sehr leicht in die Sulfoxide umgewandelt. In ähnlicher Weise gelangt man zu den Sulfoxiden durch Behandeln der Phenothiazine mit Sulfurylchlorid, z. B. in $CH_2Cl_2$ in Gegenwart von feuchtem Kieselgel bei Temperaturen zwischen etwa 0 und 30°C, vorzugsweise um 10°C.

Es ist auch möglich, eventuell so erhaltene Sulfoxide unter kräftigeren Bedingungen zu den Sulfonen zu oxydieren, wobei die Sulfoxide nicht isoliert zu werden brauchen.

Eine so erhaltene Base der allgemeinen Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z. B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diäthylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Äthansulfonsäure, Äthandisulfonsäure, 2-Hydroxyäthansulfonsäure, Benzolsulfonsäure, p-Toluolsulfonsäure, Naphthalin-mono- und -disulfonsäuren, Laurylschwefelsäure.

Die freien Basen der allgemeinen Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Gegenstand der Erfindung ist ferner die Verwendung der Verbindungen der allgemeinen Formel I und ihrer physiologisch unbedenklichen Salze zur Herstellung pharmazeutischer Zubereitungen, insbesondere auf nicht-chemischem Wege. Hierbei können sie zusammen mit mindestens einem Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der allgemeinen Formel I und/oder eines ihrer physiologisch unbedenklichen Säureadditionssalze. Diese Zubereitungen können als Arzneimittel in der Human- oder Veterinärmedizin eingesetzt werden. Als Trägersubstanzen kommen organische oder anorganische Stoffe in Frage, die sich für die enterale (z. B. orale), parenterale oder topikale Applikation eignen und mit den neuen Verbindungen nicht reagieren, beispielsweise Wasser, pflanzliche Öle, Benzylalkohole, Polyäthylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topikale Anwendung Salben, Cremes oder Puder. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z. B. ein oder mehrere Vitamine.

Man kann die Verbindungen der allgemeinen Formel I und ihre physiologisch unbedenklichen Säureadditionssalze verwenden bei der therapeutischen Behandlung des menschlichen oder tierischen Körpers und bei der Bekämpfung von Krankheiten, insbesondere von allen Formen der Hypertonie, ferner von kardialen, nephrogenen oder hepatogenen Ödemen, Aszites, Transsudaten, Schwangerschaftsödemen, Adipositas mit Flüssigkeitsretention, prämenstruellen und lokalisierten Ödemen, z. B. bei Thrombophlebitiden und auch zur Vorbeugung von Steinbildungen in den Harnwegen. Dabei werden die erfindungsgemäßen Substanzen in der Regel in Analogie zu bekannten, im Handel befindlichen Präparaten ähnlicher Indikation (z. B. Trichlormethiazid oder Hydrochlorothiazid) verabreicht, vorzugsweise in Dosierungen zwischen etwa 1 und 100 mg, insbesondere zwischen 5 und 50 mg pro Dosierungseinheit. Die tägliche Dosierung liegt vorzugsweise zwischen etwa 0,02 und 5 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist

bevorzugt.

Jede der in den folgenden Beispielen genannten Verbindungen der allgemeinen Formel I ist zur Herstellung von pharmazeutischen Zubereitungen besonders geeignet.

In den nachfolgenden Beispielen bedeutet »übliche Aufarbeitung«:

Man gibt, falls erforderlich, Wasser oder verdünnte Natronlauge hinzu, extrahiert mit einem organischen Lösungsmittel wie Chloroform, trennt ab, dampft den organischen Extrakt ein und reinigt durch Chromatographie und/oder Kristallisation die Base oder eines ihrer Salze. Temperaturen sind in Celsiusgraden angegeben.

Beispiel 1

Ein Gemisch von 68 g (0,2 Mol) 2-Chlor-10-(3-chlorpropionyl)-phenothiazin-5-oxid [erhältlich durch 12stündiges Kochen von 2-Chlorphenothiazin mit 3-Chlorpropionylchlorid in Benzol und Oxydation des erhaltenen 2-Chlor-10-(3-chlorpropionyl)-phenothiazins (F. 110–112°) mit $H_2O_2$] und 68 g (1 Mol) Imidazol wird 30 Minuten bei 110–115° gerührt. Nach üblicher Aufarbeitung erhält man 2-Chlor-10-[3-(1-imidazolyl)-propionyl]-phenothiazin-5-oxid, F. 148–149°, Hydrochlorid, F. 222–223°.

Beispiel 2

Analog Beispiel 1 erhält man aus 2-Chlor-10-(3-p-toluolsulfonyloxy-propionyl)-phenothiazin und 2-Methylimidazol das 2-Chlor-10-[3-(2-methyl-1-imidazolyl)-propionyl]-phenothiazin; Hydrochlorid, F. 258–259°.

Beispiele 3 bis 45

Analog Beispiel 1 erhält man aus den entsprechenden 10-(Chlor-acyl)- oder 10-(Brom-acyl)-phenothiazinderivaten und den entsprechenden Basen der allgemeinen Formel III:

3. 10-(1-Imidazolyl-acetyl)-phenothiazin.
4. 10-(2-Methyl-1-imidazolyl-acetyl)-phenothiazin.
5. 10-(1-Pyrazolyl-acetyl)-phenothiazin.
6. 10-(1-Benzimidazolyl-acetyl)-phenothiazin.
7. 10-[3-(1-Imidazolyl)-propionyl]-phenothiazin, F. 150–152°.
8. 10-[3-(2-Methyl-1-imidazolyl)-propionyl]-phenothiazin.
9. 10-[3-(1-Pyrazolyl)-propionyl]-phenothiazin.
10. 10-[3-(1-Benzimidazolyl)-propionyl]-phenothiazin.
11. 10-[4-(1-Imidazolyl)-butyryl]-phenothiazin.
12. 10-[4-(2-Methyl-1-imidazolyl)-butyryl]-phenothiazin.
13. 10-[4-(1-Pyrazolyl)-butyryl]-phenothiazin.
14. 10-[4-(1-Benzimidazolyl)-butyryl]-phenothiazin.
15. 2-Fluor-10-[3-(1-imidazolyl)-propionyl]-phenothiazin, Hydrochlorid, F. 244–246°.
16. 3-Fluor-10-[3-(1-imidazolyl)-propionyl]-phenothiazin, Hydrochlorid, F. 245–247°.
17. 1-Chlor-10-[3-(1-imidazolyl)-propionyl]-phenothiazin.
18. 2-Chlor-10-(1-imidazolyl-acetyl)-phenothiazin, F. 215–217°.
19. 2-Chlor-10-(2-methyl-1-imidazolyl-acetyl)-phenothiazin.
20. 2-Chlor-10-(1-pyrazolyl-acetyl)-phenothiazin.
21. 2-Chlor-10-(1-benzimidazolyl-acetyl)-phenothiazin.
22. 2-Chlor-10-[3-(1-imidazolyl)-propionyl]-phenothiazin, F. 195–196°, Hydrochlorid, F. 238–239° (Zersetzung).
23. 2-Chlor-10-[3-(1-pyrazolyl)-propionyl]-phenothiazin, Hydrochlorid, F. 157°.
24. 2-Chlor-10-[3-(1-benzimidazolyl)-propionyl]-phenothiazin, Hydrochlorid, F. 232–233°.
25. 2-Chlor-10-[4-(1-imidazolyl)-butyryl]-phenothiazin, F. 102–104°.
26. 2-Chlor-10-[4-(2-methyl-1-imidazolyl)-butyryl]-phenothiazin.
27. 2-Chlor-10-[4-(1-pyrazolyl)-butyryl]-phenothiazin.
28. 2-Chlor-10-[4-(1-benzimidazolyl)-butyryl]-phenothiazin.
29. 3-Chlor-10-[3-(1-imidazolyl)-propionyl]-phenothiazin, Hydrochlorid, F. 232–234° (Zersetzung).
30. 4-Chlor-10-[3-(1-imidazolyl)-propionyl]-phenothiazin.

31. 2-Brom-10-[3-(1-imidazolyl)-propionyl]-phenothiazin, Hydrochlorid, F. 228–230°.
32. 2-Jod-10-[3-(1-imidazolyl)-propionyl]-phenothiazin.
33. 2-Methyl-10-[3-(1-imidazolyl)-propionyl]-phenothiazin, Hydrochlorid, F. 230° (Zersetzung).
34. 3-Methyl-10-[3-(1-imidazolyl)-propionyl]-phenothiazin, Hydrochlorid, F. 228–231° (Zersetzung).
35. 2-Trifluormethyl-10-(1-imidazolyl-acetyl)-phenothiazin, Hydrochlorid, F. 178–180°.
36. 2-Trifluormethyl-10-[3-(1-imidazolyl)-propionyl]-phenothiazin, F. 126–128°.
37. 2-Trifluormethyl-10-[4-(1-imidazolyl)-butyryl]-phenothiazin.
38. 2-Cyan-10-[3-(1-imidazolyl)-propionyl]-phenothiazin, Hydrochlorid, F. 260–263°.
39. 2-Methoxy-10-[3-(1-imidazolyl)-propionyl]-phenothiazin, Hydrochlorid, F. 203–205°.
40. 3-Methoxy-10-(1-imidazolyl-acetyl)-phenothiazin.
41. 3-Methoxy-10-[3-(1-imidazolyl)-propionyl]-phenothiazin, F. 148–150°.
42. 3-Methoxy-10-[4-(1-imidazolyl)-butyryl]-phenothiazin.
43. 2-Acetyl-10-(1-imidazolyl-acetyl)-phenothiazin.
44. 2-Acetyl-10-[3-(1-imidazolyl)-propionyl]-phenothiazin, F. 106–108°.
45. 2-Acetyl-10-[4-(1-imidazolyl)-butyryl]-phenothiazin.

### Beispiel 46

Zu einem Gemisch aus 35,6 g 2-Chlor-10-[3-(1-imidazolyl)-propionyl]-phenothiazin, 8,2 g Kieselgel, 8,2 ml Wasser und 850 ml $CH_2Cl_2$ läßt man bei 10° eine Lösung von 14 g $SO_2Cl_2$ in 65 ml $CH_2Cl_2$ innerhalb von 1 Stunde zutropfen. Man rührt noch 2 Stunden bei 10°, tropft 100 ml Wasser zu und macht durch Zugabe von $NaHCO_3$ alkalisch. Nach üblicher Aufarbeitung erhält man 2-Chlor-10-[3-(1-imidazolyl)-propionyl]-phenothiazin-5-oxid, F. 148–149°.

### Beispiele 47 bis 89

Analog Beispiel 46 erhält man durch Oxydation der entsprechenden Phenothiazine:

47. 10-(1-Imidazolyl-acetyl)-phenothiazin-5-oxid.
48. 10-(2-Methyl-1-imidazolyl-acetyl)-phenothiazin-5-oxid.
49. 10-(1-Pyrazolyl-acetyl)-phenothiazin-5-oxid.
50. 10-(1-Benzimidazolyl-acetyl)-phenothiazin-5-oxid.
51. 10-[3-(1-Imidazolyl)-propionyl]-phenothiazin-5-oxid, F. 152–153°.
52. 10-[3-(2-Methyl-1-imidazolyl)-propionyl]-phenothiazin-5-oxid.
53. 10-[3-(1-Pyrazolyl)-propionyl]-phenothiazin-5-oxid.
54. 10-[3-(1-Benzimidazolyl)-propionyl]-phenothiazin-5-oxid.
55. 10-[4-(1-Imidazolyl)-butyryl]-phenothiazin-5-oxid.
56. 10-[4-(2-Methyl-1-imidazolyl)-butyryl]-phenothiazin-5-oxid.
57. 10-[4-(1-Pyrazolyl)-butyryl]-phenothiazin-5-oxid.
58. 10-[4-(1-Benzimidazolyl)-butyryl]-phenothiazin-5-oxid.
59. 2-Fluor-10-[3-(1-imidazolyl)-propionyl]-phenothiazin-5-oxid, Fumarat, F. 95–97°.
60. 3-Fluor-10-[3-(1-imidazolyl)-propionyl]-phenothiazin-5-oxid, Hydrochlorid, F. 231–233°.
61. 1-Chlor-10-[3-(1-imidazolyl)-propionyl]-phenothiazin-5-oxid.
62. 2-Chlor-10-(1-imidazolyl-acetyl)-phenothiazin-5-oxid, F. 162–163°.
63. 2-Chlor-10-(2-methyl-imidazolyl-acetyl)-phenothiazin-5-oxid.
64. 2-Chlor-10-(1-pyrazolyl-acetyl)-phenothiazin-5-oxid.
65. 2-Chlor-10-(1-benzimidazolyl-acetyl)-phenothiazin-5-oxid.
66. 2-Chlor-10-[3-(2-methyl-1-imidazolyl)-propionyl]-phenothiazin-5-oxid, Hydrochlorid, F. 222–223°.
67. 2-Chlor-10-[3-(1-pyrazolyl)-propionyl]-phenothiazin-5-oxid, F. 156–157°.
68. 2-Chlor-10-[3-(1-benzimidazolyl)-propionyl]-phenothiazin-5-oxid, F. 196–197°.
69. 2-Chlor-10-[4-(1-imidazolyl)-butyryl]-phenothiazin-5-oxid.
70. 2-Chlor-10-[4-(2-methyl-1-imidazolyl)-butyryl]-phenothiazin-5-oxid.
71. 2-Chlor-10-[4-(1-pyrazolyl)-butyryl]-phenothiazin-5-oxid.
72. 2-Chlor-10-[4-(1-benzimidazolyl)-butyryl]-phenothiazin-5-oxid.

73. 3-Chlor-10-[3-(1-imidazolyl)-propionyl]-phenothiazin-5-oxid, Hydrochlorid, F. 212 – 215° (Zersetzung).
74. 4-Chlor-10-[3-(1-imidazolyl)-propionyl]-phenothiazin-5-oxid.
75. 2-Brom-10-[3-(1-imidazolyl)-propionyl]-phenothiazin-5-oxid.
76. 2-Jod-10-[3-(1-imidazolyl)-propionyl]-phenothiazin-5-oxid.
77. 2-Methyl-10-[3-(1-imidazolyl)-propionyl]-phenothiazin-5-oxid, Hydrochlorid, F. 210 – 212°.
78. 3-Methyl-10-[3-(1-imidazolyl)-propionyl]-phenothiazin-5-oxid, Hydrochlorid, F. 224 – 226° (Zersetzung).
79. 2-Trifluormethyl-10-(1-imidazolyl-acetyl)-phenothiazin-5-oxid.
80. 2-Trifluormethyl-10-[3-(1-imidazolyl)-propionyl]-phenothiazin-5-oxid, Fumarat, F. 140° (Zersetzung).
81. 2-Trifluormethyl-10-[4-(1-imidazolyl)-butyryl]-phenothiazin-5-oxid.
82. 2-Cyan-10-[3-(1-imidazolyl)-propionyl]-phenothiazin-5-oxid, F. 127 – 129°, Hydrochlorid, F. 208 – 210° (Zersetzung).
83. 2-Methoxy-10-[3-(1-imidazolyl)-propionyl]-phenothiazin-5-oxid, Fumarat, F. 90 – 94°.
84. 3-Methoxy-10-(1-imidazolyl-acetyl)-phenothiazin-5-oxid.
85. 3-Methoxy-10-[3-(1-imidazolyl)-propionyl]-phenothiazin-5-oxid, Fumarat, F. 89 – 92°.
86. 3-Methoxy-10-[4-(1-imidazolyl)-butyryl]-phenothiazin-5-oxid.
87. 2-Acetyl-10-(1-imidazolyl-acetyl)-phenothiazin-5-oxid.
88. 2-Acetyl-10-[3-(1-imidazolyl)-propionyl]-phenothiazin-5-oxid, Fumarat, F. 159 – 162° (Zersetzung).
89. 2-Acetyl-10-[4-(1-imidazolyl)-butyryl]-phenothiazin-5-oxid.

## Beispiel 90

Man rührt ein Gemisch von 19,9 g Phenothiazin, 14 g 3-(1-Imidazolyl)-propionsäure, 20,6 g Dicyclohexylcarbodiimid und 200 ml Äthylacetat 30 Minuten bei 0°, dann 3 Stunden bei 20°, arbeitet wie üblich auf und erhält 10-[3-(1-Imidazolyl)-propionyl]-phenothiazin, F. 150 – 152°.

## Beispiel 91

Man rührt ein Gemisch von 25 g 2-Chlor-phenothiazin-5-oxid, 14 g 3-(1-Imidazolyl)-propionsäure, 20,6 g Dicyclohexylcarbodiimid und 250 ml Tetrahydrofuran 1 Stunde bei 0°, dann 2 Stunden bei 30°, arbeitet wie üblich auf und erhält 2-Chlor-10-[3-(1-imidazolyl)-propionyl]-phenothiazin-5-oxid, F. 148 – 149°.

## Beispiel 92

Man rührt ein Gemisch von 1 g 2-Chlor-10-[3-(1-imidazolyl)-propionyl]-phenothiazin, 8 ml 30%iges $H_2O_2$ und 40 ml Essigsäure 3 Stunden bei 45°, arbeitet wie üblich auf und erhält 2-Chlor-10-[3-(1-imidazolyl)-propionyl]-phenothiazin-5,5-dioxid, F. 134 – 135° (Zersetzung).

## Beispiele 93 bis 135

Analog Beispiel 92 erhält man durch Oxydation der entsprechenden Phenothiazine:

93. 10-(1-Imidazolyl-acetyl)-phenothiazin-5,5-dioxid.
94. 10-(2-Methyl-1-imidazolyl-acetyl)-phenothiazin-5,5-dioxid.
95. 10-(1-Pyrazolyl-acetyl)-phenothiazin-5,5-dioxid.
96. 10-(1-Benzimidazolyl-acetyl)-phenothiazin-5,5-dioxid.
97. 10-[3-(1-Imidazolyl)-propionyl]-phenothiazin-5,5-dioxid.
98. 10-[3-(2-Methyl-1-imidazolyl)-propionyl]-phenothiazin-5,5-dioxid.
99. 10-[3-(1-Pyrazolyl)-propionyl]-phenothiazin-5,5-dioxid.
100. 10-[3-(1-Benzimidazolyl)-propionyl]-phenothiazin-5,5-dioxid.
101. 10-[4-(1-Imidazolyl)-butyryl]-phenothiazin-5,5-dioxid.
102. 10-[4-(2-Methyl-1-imidazolyl)-butyryl]-phenothiazin-5,5-dioxid.
103. 10-[4-(1-Pyrazolyl)-butyryl]-phenothiazin-5,5-dioxid.
104. 10-[4-(1-Benzimidazolyl)-butyryl]-phenothiazin-5,5-dioxid.

105. 2-Fluor-10-[3-(1-imidazolyl)-propionyl]-phenothiazin-5,5-dioxid.
106. 3-Fluor-10-[3-(1-imidazolyl)-propionyl]-phenothiazin-5,5-dioxid.
107. 1-Chlor-10-[3-(1-imidazolyl)-propionyl]-phenothiazin-5,5-dioxid.
108. 2-Chlor-10-(1-imidazolyl-acetyl)-phenothiazin-5,5-dioxid.
109. 2-Chlor-10-(2-methyl-imidazolyl-acetyl)-phenothiazin-5,5-dioxid.
110. 2-Chlor-10-(1-pyrazolyl-acetyl)-phenothiazin-5,5-dioxid.
111. 2-Chlor-10-(1-benzimidazolyl-acetyl)-phenothiazin-5,5-dioxid.
112. 2-Chlor-10-[3-(2-methyl-1-imidazolyl)-propionyl]-phenothiazin-5,5-dioxid, Hydrochlorid, F. 224° (Zersetzung).
113. 2-Chlor-10-[3-(1-pyrazolyl)-propionyl]-phenothiazin-5,5-dioxid.
114. 2-Chlor-10-[3-(1-benzimidazolyl)-propionyl]-phenothiazin-5,5-dioxid.
115. 2-Chlor-10-[4-(1-imidazolyl)-butyryl]-phenothiazin-5,5-dioxid.
116. 2-Chlor-10-[4-(2-methyl-1-imidazolyl)-butyryl]-phenothiazin-5,5-dioxid.
117. 2-Chlor-10-[4-(1-pyrazolyl)-butyryl]-phenothiazin-5,5-dioxid.
118. 2-Chlor-10-[4-(1-benzimidazolyl)-butyryl]-phenothiazin-5,5-dioxid.
119. 3-Chlor-10-[3-(1-imidazolyl)-propionyl]-phenothiazin-5,5-dioxid.
120. 4-Chlor-10-[3-(1-imidazolyl)-propionyl]-phenothiazin-5,5-dioxid.
121. 2-Brom-10-[3-(1-imidazolyl)-propionyl]-phenothiazin-5,5-dioxid.
122. 2-Jod-10-[3-(1-imidazolyl)-propionyl]-phenothiazin-5,5-dioxid.
123. 2-Methyl-10-[3-(1-imidazolyl)-propionyl]-phenothiazin-5,5-dioxid.
124. 3-Methyl-10-[3-(1-imidazolyl)-propionyl]-phenothiazin-5,5-dioxid.
125. 2-Trifluormethyl-10-(1-imidazolyl-acetyl)-phenothiazin-5,5-dioxid.
126. 2-Trifluormethyl-10-[3-(1-imidazolyl)-propionyl]-phenothiazin-5,5-dioxid.
127. 2-Trifluormethyl-10-[4-(1-imidazolyl)-butyryl]-phenothiazin-5,5-dioxid.
128. 2-Cyan-10-[3-(1-imidazolyl)-propionyl]-phenothiazin-5,5-dioxid, F. 139–142° (Zersetzung), Hydrochlorid, F. 220° (Sintern ab 215°).
129. 2-Methoxy-10-[3-(1-imidazolyl)-propionyl]-phenothiazin-5,5-dioxid.
130. 3-Methoxy-10-(1-imidazolyl-acetyl)-phenothiazin-5,5-dioxid.
131. 3-Methoxy-10-[3-(1-imidazolyl)-propionyl]-phenothiazin-5,5-dioxid, Hydrochlorid, F. 125° (Zersetzung).
132. 3-Methoxy-10-[4-(1-imidazolyl)-butyryl]-phenothiazin-5,5-dioxid.
133. 2-Acetyl-10-(1-imidazolyl-acetyl)-phenothiazin-5,5-dioxid.
134. 2-Acetyl-10-[3-(1-imidazolyl)-propionyl]-phenothiazin-5,5-dioxid.
135. 2-Acetyl-10-[4-(1-imidazolyl)-butyryl]-phenothiazin-5,5-dioxid.

### Beispiele 136 bis 222

Analog Beispiel 1 erhält man aus den entsprechenden 10-(Chlor-acyl)- oder 10-(Brom-acyl)-phenothiazin-5-oxiden bzw. aus den entsprechenden 10-(Chlor-acyl)- oder 10-(Brom-acyl)-phenothiazin-5,5-dioxiden durch Reaktion mit den entsprechenden Basen der allgemeinen Formel III die in den Beispielen 47 bis 89 beschriebenen Sulfoxide bzw. die in den Beispielen 92 bis 135 beschriebenen Sulfone.

### Beispiele 223 bis 352

Analog Beispiel 90 oder 91 erhält man aus den entsprechenden Phenothiazinen, Phenothiazin-5-oxiden und Phenothiazin-5,5-dioxiden der allgemeinen Formel IV mit den entsprechenden Carbonsäuren der allgemeinen Formel V die in den Beispielen 2 bis 6, 8 bis 45, 47 bis 89 und 92 bis 136 beschriebenen Verbindungen.

### Beispiel 353

Man rührt ein Gemisch von 1 g 2-Chlor-10-[3-(1-imidazolyl)-propionyl]-phenothiazin-5-oxid, 4 ml 30%iges $H_2O_2$ und 40 ml Essigsäure 3 Stunden bei 45°, arbeitet wie üblich auf und erhält 2-Chlor-10-[3-(1-imidazolyl)-propionyl]-phenothiazin-5,5-dioxid, F. 134–135° (Zersetzung), Hydrochlorid, F. 200–201°.

Die nachstehenden Beispiele betreffen pharmazeutische Zubereitungen, die Verbindungen der allgemeinen Formel I enthalten:

Beispiel A

Tabletten

Ein Gemisch von 1 kg 2-Chlor-10-[3-(1-imidazolyl)-propionyl]-phenothiazin-5,5-dioxid, 4 kg Lactose, 1,2 kg Maisstärke, 0,2 kg Talk und 0,1 kg Magnesiumstearat wird in üblicher Weise zu Tabletten gepreßt, derart, daß jede Tablette 10 mg Wirkstoff enthält.

Beispiel B

Dragees

Analog Beispiel A werden Tabletten gepreßt, die anschließend in üblicher Weise mit einem Überzug aus Saccharose, Weizenstärke, Talk, Tragant und Farbstoff überzogen werden.

Beispiel C

Kapseln

5 kg 2-Chlor-10-[3-(1-imidazolyl)-propionyl]-phenothiazin-5,5-dioxid werden in üblicher Weise in Hartgelatinekapseln gefüllt, so daß jede Kapsel 20 mg des Wirkstoffes enthält.

Analog sind Tabletten, Dragees und Kapseln erhältlich, die einen oder mehrere der übrigen Wirkstoffe der allgemeinen Formel I und/oder ihre physiologisch unbedenklichen Säureadditionssalze enthalten.

**Patentansprüche**

1. Phenothiazinderivate der allgemeinen Formel I

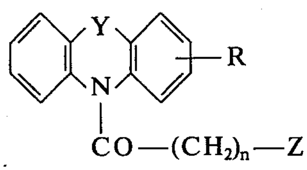

(I)

worin

R H, F, Cl, Br, J, $CH_3$, $CF_3$, CN, $CH_3O$ oder $CH_3CO$,
Y S, SO oder $SO_2$,
Z 1-Imidazolyl, 2-Methyl-1-imidazolyl, 1-Pyrazolyl oder 1-Benzimidazolyl und
n 1, 2 oder 3 bedeuten,

und deren physiologisch unbedenkliche Säureadditionssalze.

2. a) 2-Chlor-10-[3-(1-imidazolyl)-propionyl]-phenothiazin;
b) 2-Chlor-10-[3-(1-imidazolyl)-propionyl]-phenothiazin-5-oxid;
c) 2-Chlor-10-[3-(1-imidazolyl)-propionyl]-phenothiazin-5,5-dioxid.

3. Verfahren zur Herstellung von Phenothiazinderivaten der allgemeinen Formel I

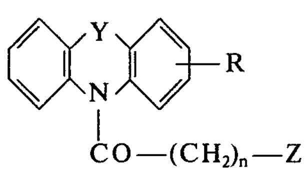

(I)

worin

R H, F, Cl, Br, J, $CH_3$, $CF_3$, CN, $CH_3O$ oder $CH_3CO$,
Y S, SO oder $SO_2$,
Z 1-Imidazolyl, 2-Methyl-1-imidazolyl, 1-Pyrazolyl oder 1-Benzimidazolyl und
n 1, 2 oder 3 bedeuten,

sowie von deren physiologisch unbedenklichen Säureadditionssalzen, dadurch gekennzeichnet, daß man ein Phenothiazinderivat der allgemeinen Formel II

$$Phe—CO—(CH_2)_n—X \qquad (II)$$

worin

Phe den Rest

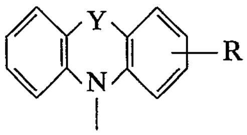

und

X Cl, Br, J, OH oder reaktionsfähig verestertes OH bedeutet und

R, Y und n die angegebene Bedeutung haben

mit einer Base der allgemeinen Formel III

$$H—Z \qquad (III)$$

worin

Z die angegebene Bedeutung hat

oder daß man ein Phenothiazinderivat der allgemeinen Formel IV

$$Phe—H \qquad (IV)$$

worin

Phe die angegebene Bedeutung hat

mit einer Carbonsäure der allgemeinen Formel V

$$HOOC—(CH_2)_n—Z \qquad (V)$$

worin

n und Z die angegebene Bedeutung haben,

oder einem ihrer funktionellen Derivate umsetzt und daß man gegebenenfalls durch Behandeln mit oxydierenden Mitteln ein so erhaltenes Phenothiazin der allgemeinen Formel I (Y=S) zum Sulfoxid oder Sulfon der allgemeinen Formel I (Y=SO oder $SO_2$) oder ein so erhaltenes Sulfoxid der allgemeinen Formel I (Y=SO) zum Sulfon der allgemeinen Formel I (Y=$SO_2$) oxydiert und/oder eine so erhaltene Base der allgemeinen Formel I durch Behandeln mit einer Säure in eines ihrer physiologisch unbedenklichen Säureadditionssalze umwandelt.

4. Verfahren zur Herstellung pharmazeutischer Zubereitungen, dadurch gekennzeichnet, daß man eine Verbindung der allgemeinen Formel I und/oder eines ihrer physiologisch unbedenklichen Säureadditionssalze zusammen mit mindestens einem festen, flüssigen oder halbflüssigen Träger- oder Hilfsstoff und gegebenenfalls in Kombination mit einem oder mehreren weiteren Wirkstoffen in eine geeignete Dosierungsform bringt.

5. Pharmazeutische Zubereitung, gekennzeichnet durch einen Gehalt an mindestens einer Verbindung der allgemeinen Formel I und/oder einem ihrer physiologisch unbedenklichen Säureadditionssalze.

**Claims**

1. Phenothiazine derivatives of the general formula I

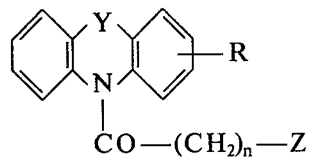

(I)

wherein

R is H, F, Cl, Br, I, $CH_3$, $CF_3$, CN, $CH_3O$ or $CH_3CO$,
Y is S, SO or $SO_2$,
Z is imidazol-1-yl, 2-methyl-imidazol-1-yl, pyrazol-1-yl or benzimidazol-1-yl and
n is 1, 2 or 3,

and physiologically acceptable acid addition salts thereof.

2. a) 2-Chloro-10-[3-(imidazol-1-yl)-propionyl]-phenothiazine;
b) 2-chloro-10-[3-(imidazol-1-yl)-propionyl]-phenothiazine-5-oxide;
c) 2-chloro-10-[3-(imidazol-1-yl)-propionyl]-phenothiazine-5,5-dioxide.

3. Process for the preparation of phenothiazine derivatives of the general formula I

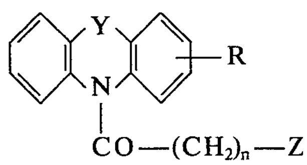

(I)

wherein

R is H, F, Cl, Br, I, $CH_3$, $CF_3$, CN, $CH_3O$ or $CH_3CO$,
Y is S, SO or $SO_2$,
Z is imidazol-1-yl, 2-methyl-imidazol-1-yl, pyrazol-1-yl or benzimidazol-1-yl and
n is 1, 2 or 3,

and of physiologically acceptable acid addition salts thereof, characterised in that a phenothiazine derivative of the general formula II

$$Phe—CO—(CH_2)_n—X \qquad (II)$$

wherein

Phe is the radical

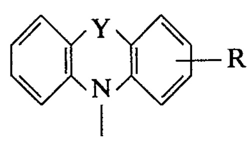

and
X is Cl, Br, I, OH or reactive esterified OH and
R, Y and n have the meaning indicated,

is reacted with a base of the general formula III

$$H—Z \qquad (III)$$

wherein

Z has the meaning indicated,

or a phenothiazine derivative of the general formula IV

$$Phe—H \qquad (IV)$$

wherein

Phe has the meaning indicated,

is reacted with a carboxylic acid of the general formula V

$$HOOC—(CH_2)_n—Z \qquad (V)$$

wherein

n and Z have the meaning indicated,

or with one of its functional derivates, and a thus obtained phenothiazine of the general formula I (Y = S) is optionally oxidised by treatment with oxidising agents to give the sulphoxide or sulphone of the general formula I (Y = SO or $SO_2$) or a thus obtained sulphoxide of the general formula I (Y = SO) is optionally oxidised by treatment with oxidising agents to give the sulphone of the general formula I (Y = $SO_2$) and/or a thus obtained base of the general formula I is converted by treatment with an acid, into one of its physiologically acceptable acid addition salts.

4. Process for the preparation of pharmaceutical formulations, characterised in that a compound of the general formula I and/or one of its physiologically acceptable acid addition salts is brought into a suitable dosage form together with at least one solid, liquid or semi-liquid excipient or auxiliary and, if appropriate, in combination with one or more further active compounds.

5. Pharmaceutical formulation, characterised in that it contains at least one compound of the general formula I and/or one of its physiologically acceptable acid addition salts.

**Revendications**

1. Dérivés de la phénothiazine de formule générale I

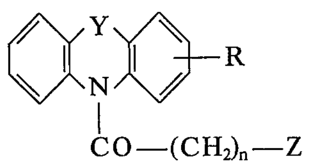

(I)

dans laquelle

R représente H, F, Cl, Br, I, $CH_3$, $CF_3$, CN, $CH_3O$ ou $CH_3CO$,
Y représente S, SO ou $SO_2$,
Z représente un groupe 1-imidazolyle, 2-méthyl-1-imidazolyle, 1-pyrazolyle ou 1-benzimidazolyle et
n est égal à 1, 2 ou 3,

et leurs sels formés par addition avec des acides tolérés par l'organisme.

2. a) La 2-chloro-10-[3-(1-imidazolyl)-propionyl]-phénothiazine;
b) Le 5-oxyde de 2-chloro-10-[3-(1-imidazolyl)-propionyl]-phénothiazine;
c) Le 5,5-dioxyde de 2-chloro-10-[3-(1-imidazolyl)-propionyl]-phénothiazine.

3. Procédé de préparation des dérivés de phénothiazine de formule générale I

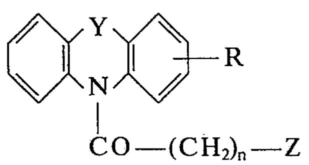

(I)

dans laquelle

R représente H, F, Cl, Br, I, $CH_3$, $CF_3$, CN, $CH_3O$ ou $CH_3CO$,
Y représente S, SO ou $SO_2$,
Z représente un groupe 1-imidazolyle, 2-méthyl-1-imidazolyle, 1-pyrazolyle ou 1-benzimidazolyle et
n est égal à 1, 2 ou 3,

et de leurs sels formés par addition avec des acides tolérés par l'organisme, caractérisé en ce que l'on fait réagir un dérivé de la phénothiazine de formule générale II

$$Phe—CO—(CH_2)_n—X \qquad (II)$$

dans laquelle

Phe représente le reste

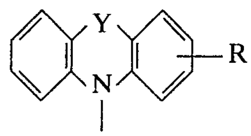

et

X représente Cl, Br, I, OH ou un groupe OH estérifié réactif et

R, Y et n ont les significations indiquées ci-dessus,

avec une base de formule générale III

$$H—Z \qquad (III)$$

dans laquelle

Z a la signification indiquée ci-dessus,

ou en ce que l'on fait réagir un dérivé de la phénothiazine de formule générale IV

$$Phe—H \qquad (IV)$$

dans laquelle

Phe a la signification indiquée ci-dessus,

avec un acide carboxylique de formule générale V

$$HOOC—(CH_2)_n—Z \qquad (V)$$

dans laquelle

n et Z ont les significations indiquées ci-dessus,

ou un dérivé fonctionnel d'un tel acide, et en ce que, le cas échéant, par traitement à l'aide d'agents oxydants, on oxyde une phénothiazine de formule générale I ($Y=S$) ainsi obtenue en sulfoxyde ou sulfone de formule générale I ($Y=SO$ ou $SO_2$) ou on oxyde un sulfoxyde de formule générale I ($Y=SO$) ainsi obtenu en sulfone de formule générale I ($Y=SO_2$) et/ou on convertit une base de formule générale I ainsi obtenue, par traitement à l'aide d'un acide, en un sel formé par addition avec un acide toléré par l'organisme.

4. Procédé de préparation de compositions pharmaceutiques, caractérisé en ce que l'on met un composé de formule générale I et/ou l'un de ses sels formés par addition avec des acides tolérés par l'organisme, avec au moins un véhicule ou produit auxiliaire et le cas échéant en combinaison avec une ou plusieurs autres substances actives, sous une forme de dosage appropriée.

5. Composition pharmaceutique, caractérisée en ce qu'elle contient au moins un composé de formule générale I et/ou l'un de ses sels formés par addition avec des acides tolérés par l'organisme.